# EUROPEAN PATENT APPLICATION

(11) **EP 2 614 845 A2**
(43) Date of publication of application: **17.07.2013**
(21) Application number: 12186538.0
(22) Date of filing: 28.09.2012
(51) Int. Cl.: A61L 31/04, A61L 31/06

(54) **Application of composition containing telechelic macromer and photoinitiator for producing implant for hernia repair**

(30) Priority: 29.09.2011 PL 39646911
(71) Applicant: Zachodniopomorski Uniwersytet Technologiczny w Szczecinie, 70-310 Szczecin (PL)
(72) Inventor: El Fray, Miroslawa, 72-003 Dobra (PL); Skrobot, Jedrzej, 14-200 Ilawa (PL); Zair, Labib, 74-100 Gryfino (PL)
(74) Representative: Malcherek, Piotr

(57) **Abstract**

The present invention relates to an application of a composition containing telechelic macromer and photoinitiator for producing implant for hernia repair. The macromer structure is defined by Formula 1:

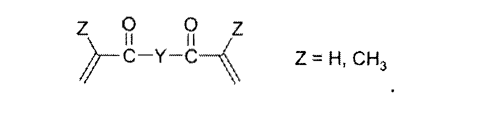

The macromer bears (meth)acrylic end-groups and comprises a core Y defined by Formulas 2 to 9, that is linked by urethane, ester or anhydride bonds to the (meth)acrylic groups. The iodine value of the macromer is from 5 to 75. The composition according to the present invention can be shaped at room temperature at 37 °C and is capable of undergoing liquid-to-solid transition upon irradiation with low intensity light. Upon irradiation with light the composition gains desired physicochemical properties and turns into an elastic solid. The implant for hernia repair serves for closing the hernia opening and for enhancing mechanical strength of abdominal wall.

## Description

The present invention relates to the application of a composition containing telechelic macromer and photoinitiator for producing implant for hernia repair. The role of the implant is to close the hernia opening and to enhance mechanical strength of abdominal wall.

There are numerous patent applications and literature reports on implants for reinforcing the area of treated hernia. These implants are in the form of a patch made of fine sheet of mesh, which in most cases is made of polypropylene. After the lining of the abdominal cavity has been cut through, this polypropylene mesh is inserted into the site of hernia opening and is sutured to deep fascia of abdominal muscles.

A method of hernia treatment with the help of polypropylene mesh has been revealed in the patent application US 5,290,217. According to this invention, the mesh is inserted into the herniated area using laparoscopic technique and is then fastened to tissues- with the use of special clips. The patent application US 5,634,931 discloses a mesh made of perlon fiber. This mesh is tightened by an elastic fiber stitched along its edges. The patent application US 6,176,863 reports on a two-layer repair patch. The first and second layers are joined together by a seam that defines a periphery of a pouch between the layers. A resilient monofilament I-shaped spring is located within the pouch at the seam for urging the patch to conform to the generally planar configuration. Polish pending patent application P395338 reports on a composition containing telechelic macromer and photoinitiator. The present invention relates to application of this composition.

The present invention relates to an application of a composition containing telechelic macromer and photoinitiator for producing implant for hernia repair. Said composition contains macromer defined by Formula 1, that is end-capped with (meth)acrylic groups and comprises a core Y defined by Formulas 2 to 9 and this core is linked by urethane, ester, or anhydride bonds to the (meth)acrylic groups. The iodine value of said macromer is in the range of 5 to 75. The composition is a viscous liquid or paste at room temperature and at 37 °C and is susceptible to external stimuli, such as UV/Vis light.

The precursor of the core Y defined by formula 2 is a branched dimer fatty acid compound of 36 carbon atoms per molecule, bearing primary amine end-groups and its amine value is in the range of 200-210 mg KOH/g.

The macromer with core Y defined by formula 2 has urethane bonds formed by a reaction of the core precursor with trimethylene carbonate or propylene carbonate and ester bonds formed by subsequent reaction with acryloyl or methacryloyl chloride.

The precursor of the core Y defined by formula 3 comprises a branched dimer fatty acid compound of 36 carbon atoms per molecule, or the precursor of the core Y defined by formula 3 comprises derivatives of this dimer fatty acid, which are the products of esterification of the dimer fatty acid compound with low molar mass diols of 2, 4 or 6 carbon atoms per molecule, of molar mass ranging from 540 to 2000 g/mol bearing hydroxyl end-groups and its hydroxyl value is in the range of 50-210 mg KOH/g, or the precursor of the core Y defined by formula 3 comprises a linear aliphatic polyether or poly(ether-ester) of molar mass in the range of 600-2500 g/mol bearing hydroxyl end-groups, or the precursor of the core Y defined by formula 3 comprises a branched polyether compound of molar mass of 1500 g/mol, bearing hydroxyl end-groups.

The macromer comprising core Y defined by formula 3 has urethane bonds formed by a reaction of a precursor with 1,6-diisocyanatohexane or with 5-isocyanato-1-(isocyanatomethyl)-1,3,3-trimethylcyclohexane, or with 1,1'-methylenebis(4-isocyanatocyclohexane).

The precursor of the core Y defined by formulas 4 or 5 is a branched dimer fatty acid compound of 36 carbon atoms per molecule, or the precursor of the core Y defined by formulas 4 or 5 is a derivative of this dimer fatty acid which is the product of esterification of the dimer fatty acid compound with low molar mass diols of 2, 4 or 6 carbon atoms per molecule, of molar mass ranging from 540 to 2000 g/mol bearing hydroxyl end-groups and its hydroxyl value is in the range of 50-210 mg KOH/g.

The precursor of the core Y defined by formula 6 is a linear aliphatic poly(ether-ester) of molar mass in the range of 600-2500 g/mol bearing hydroxyl end-groups.

The precursor of the core Y defined by formula 7 is a linear aliphatic polyether of molar mass in the range of 600-2500 g/mol bearing hydroxyl end-groups.

The precursor of the core Y defined by formula 8 is a branched polyether compound of molar mass of 1500 g/mol, bearing hydroxyl end-groups.

The telechelic macromer comprising core Y defined by formulas 4 or 5, or 6, or 7, or 8 has linking ester bonds formed by reaction of the precursor with acryloyl or methacryloyl chloride.

The precursor of the core Y defined by formula 9 comprises a branched dimer fatty acid compound of 36 carbon atoms per molecule, or the precursor of the core Y defined by formula 9 comprises derivatives of this dimer fatty acid which are the products of esterification of the dimer fatty acid compound with low molar mass diols of 2, 4 or 6 carbon atoms per molecule, of molar mass ranging from 570 to 3000 g/mol bearing carboxyl end-groups and its carboxyl value is in the range of 35-200 mg KOH/g.

The telechelic macromer with core Y defined by formula 9 comprises anhydride bonds formed by reaction of the precursor with acryloyl or methacryloyl chloride.

The photoinitiator content is in the range of 0.5-2% with respect to the total weight of the composition ( if the composition comprises only the macromer and the photoinitiator - in relation to the macromer, whereas if the composition also comprises a diluent - in relation to the macromer with the diluent ).

Preferably, the photoinitiator is a compound having in its structure an aromatic ring in position α to carbonyl group, possibly substituents at the aromatic ring or phosphine oxide group in the amount of 0.5 - 2 %. Of the total weight. For example, 2-hydroxy-1-[4-(2-hydroxyethoxy)phenyl]-2-methyl-1-propanone or phenylbis (2,4,6-trimethylbenzoyl)phosphine oxide, or 2,2-Dimethoxy-1,2-diphenylethan-1-one can be used.

The composition may comprise a reactive diluent, poly(ethylene glycol) diacrylate of molar mass in the range of 250-700 g/mol. The weight content of the reactive diluent in the composition varies from 1:99 to 99:1.

The composition may comprise one or more of the three reactive diluents, trimethylolpropane triacrylate or tri(propylene glycol) diacrylate or ethoxylated trimethylolpropane triacrylate. The weight content of the reactive diluent in the composition varies from 1:99 to 99:1. The composition comprising one or more of the three listed diluents may, additionally, comprise poly(ethylene glycol) diacrylate of molar mass in the range of 250-700 g/mol.

The composition may contain powder fillers such as sodium chloride, or sucrose, or a polysaccharide compound of grain size varying between 100 and 300 micrometers, where the filler weight content in the composition is between 30 and 80%. These fillers serve as porogens.

After the composition has been injected, it is irradiated with ITV/VIS light of wavelength above 280 nm and of intensity below 50 mW/cm², which causes its cross-linking.

The composition can be injected directly as a viscous liquid or paste into the herniated area or the implant can be shaped in sterile conditions outside human body and then can be administered to the patient. In case the composition is injected into the herniated area, the viscous paste is shaped in such a manner-that its edges extend beyond the edges of the hernia opening.

An advantage of the present invention is that the implant shape perfectly matches the shape of hernia opening and its edges extend beyond the edges of the hernia opening. An advantage of applying said composition for producing implant is that the implant undergoes biodegradation *in vivo.* As a result of applying said composition, the time of transition from viscous liquid to hernia implant is less than 5 minutes. Another advantage of the present invention is that injecting said composition requires only slight incision of lining of the abdominal cavity, which contributes to minimizing complications after a surgery. The present invention allows for the injection and for producing implant within a short timespan, which significantly shortens the time of the surgery and the overall time of treatment.

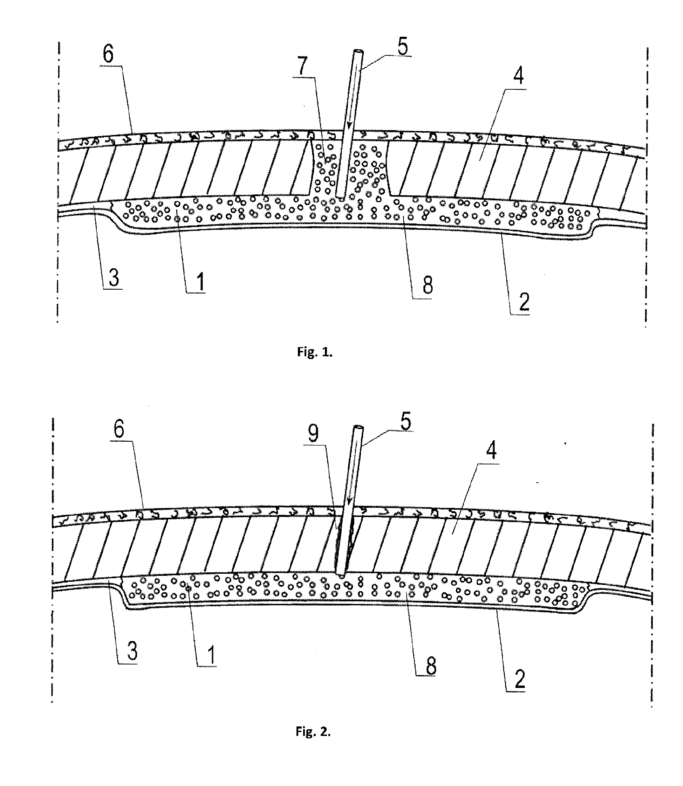

The present invention is illustrated in the following Examples, formulas and in the Figures. Formula 1 shows telechelic macromer, formula 2 shows core of macromer comprising linking ester and urethane groups, formula 3 shows another core of macromer comprising substituents X and Rₙ, formula 4 shows another core of macromer comprising linking ester bonds, formula 5 shows another core of macromer comprising linking ester bonds, formula 6 shows another core of macromer comprising linking ester bonds, formula 7 shows another core of macromer comprising linking ester bonds, formula 8 shows another core of macromer comprising linking ester bonds, formula 9 shows core of macromer comprising linking anhydride bonds. Figure 1 represents cross-section of lining of the abdominal cavity in the hernia region at the moment of injecting the composition for producing hernia implant, in the case when the hernia opening is not closed with stitches. Figure 2 represents cross-section of lining of the abdominal cavity in the hernia region at the moment of injecting the composition for producing hernia implant, in the case when the hernia opening is closed with stitches.

### Example I

A pasty composition containing telechelic macromer and photoinitiator is injected into the gap 1 between peritoneum 2 and deep fascia 3 of abdominal muscles 4 by means of a pipe 5. The composition is injected into gap 1 through small opening in skin 6, thus filling the gap 1 and the inside of hernia opening 7, thus forming patch 8 of 1-3 mm in thickness. The size of the patch, which implies the amount of injected composition, is larger than the hernia opening. The strip between the edge of implant and the edge of hernia opening is 5 to 15 mm wide.

After the patch has been formed, the composition is irradiated with UV/Vis light of wavelength above 280 nm and total intensity of 20 mW/cm², during 100 seconds. The composition is cross-linked and an elastic solid patch obtained.

### Example II

The procedure is performed in a way analogous to Example I, with the difference that the hernia opening is closed with stitches 9.

### Example III

A patch is formed on a Petri dish in sterile conditions outside human body from a composition containing telechelic macromer and photoinitiator at room temperature. The patch has a round or elliptical shape, thickness of 1 to 3 mm and a diameter of 1 to 7 cm. The composition is irradiated with UV/Vis light of wavelength above 280 nm and total intensity of 10 mW/cm², during 200 seconds. The composition is cross-linked and an elastic solid patch is obtained. The obtained patch is rolled up to form a roll of appropriate diameter, allowing for delivering it with an applicator. The patch is delivered to the area of hernia opening which is closed with stitches 9. Then the patch is unrolled on the face of closed hernia opening and is fastened by absorbable sutures.

## Claims

1. Application of composition containing telechelic macromer and photoinitiator, wherein macromer defined by formula 1 bears (meth)acrylic end-groups and comprises core Y defined by formulas 2 to 9 that is linked by urethane, ester or anhydride groups to the (meth)acrylic groups, and wherein the macromer has iodine value in the range of 5 to 75, for producing implant for hernia repair.

2. Application according to claim 1, wherein the precursor of the core defined by formula 2 is a branched dimer fatty acid compound of 36 carbon atoms per molecule, bearing primary amine end-groups and its amine value is in the range of 200-210 mg KOH/g.

3. Application according to claim 2, wherein telechelic macromer comprises urethane bonds formed by reaction of the core precursor with trimethylene carbonate or propylene carbonate and linking ester bonds formed by subsequent reaction with acryloyl or methacryloyl chloride.

4. Application according to claim 1, wherein the precursor of the core defined by formula 3 comprises a branched dimer fatty acid compound of 36 carbon atoms per molecule or it comprises derivatives of this dimer fatty acid, which are the products of esterification of the dimer fatty acid compound with low molar mass diols of 2, 4 or 6 carbon atoms per molecule, of molar mass ranging from 540 to 2000 g/mol bearing hydroxyl end-groups and its hydroxyl value is in the range of 50-210 mg KOH/g, or wherein the precursor of the core Y defined by formula 3 comprises a linear aliphatic polyether or poly(ether-ester) of molar mass in the range of 600-2500 g/mol bearing hydroxyl end-groups, or wherein the precursor of the core Y defined by formula 3 comprises a branched polyether compound of molar mass of 1500 g/mol, bearing hydroxyl end-groups.

5. Application according to claim 4, wherein the linking urethane bonds of the macromer are formed by reaction of the core precursor with 1,6-diisocyanatohexane or with 5-isocyanato-1-(isocyanatomethyl)-1,3,3-trimethylcyclohexane, or with 1,1'-methylenebis(4-isocyanatocyclohexane).

6. Application according to claim 1, wherein the precursor of the core defined by formulas 4 and 5 comprises a branched dimer fatty acid compound of 36 carbon atoms per molecule or it comprises derivatives of this dimer fatty acid which are the products of esterification of the dimer fatty acid compound with low molar mass diols of 2, 4 or 6 carbon atoms per molecule, of molar mass ranging from 540 to 2000 g/mol bearing hydroxyl end-groups and its hydroxyl value is in the range of 50-210 mg KOH/g.

7. Application according to claim 1, wherein the precursor of the core defined by formula 6 comprises a linear aliphatic poly(ether-ester) of molar mass in the range of 600-2500 g/mol bearing hydroxyl end-groups.

8. Application according to claim 1, wherein the precursor of the core defined by formula 7 comprises a linear polyether of molar mass in the range of 600-2500 g/mol bearing hydroxyl end-groups.

9. Application according to claim 1, wherein the precursor of the core defined by formula 8 comprises a branched polyether of molar mass of 1500 g/mol bearing hydroxyl end-groups.

10. Application according to claims 6 or 7 or 8 or 9, wherein telechelic macromer comprises linking ester bonds that are formed by a reaction of the core precursor with (meth)acryloyl chloride.

11. Application according to claim 1, wherein a precursor of the core defined by formula 9 comprises branched dimer fatty acid compound of 36 carbon atoms per molecule, or it comprises derivatives of this dimer fatty acid, which are the products of esterification of the dimer fatty acid compound with low molar mass diols of 2, 4 or 6 carbon atoms per molecule, of molar mass ranging from 570 to 3000 g/mol bearing carboxyl end-groups and its carboxyl value is in the range of 35-210 mg KOH/g.

12. Application according to claim 11, wherein the linking anhydride bonds are formed by a reaction of the core precursor with (meth)acryloyl chloride.

13. Application according to claim 1, wherein the photoinitiator compound comprises an aromatic ring in position α to a carbonyl group, possibly substituents at the aromatic ring and possibly a phosphine oxide group, and wherein the amount of the photoinitiator used is of 0.5-2 weight %.

14. Application according to claim 1, wherein the composition comprises a reactive diluent which is poly(ethylene glycol) diacrylate of molar mass in the range of 250 to 700 g/mol, and wherein the weight content of the reactive diluent in the composition varies from 1:99 to 99:1.

15. Application according to claims 1 or 14, wherein the composition comprises a reactive diluent which is one or more of the following: trimethylolpropane triacrylate or tri(propylene glycol) diacrylate or ethoxylated trimethylolpropane triacrylate, and wherein the weight content of the reactive diluent in the composition varies from 1:99 to 99:1.

16. Application according to claims 1, wherein the composition comprises powder fillers such as sodium chloride, or sucrose, or polysaccharide compound with grain size varying between 100 and 300 micrometers, and wherein the filler weight content in the composition is between 30 and 80%. 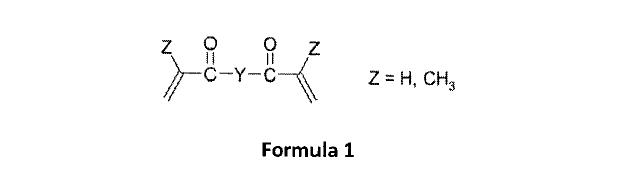 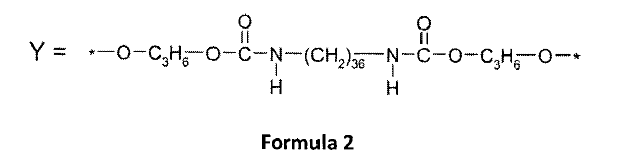 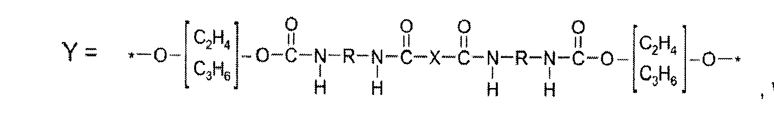 where 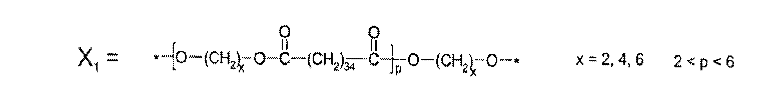 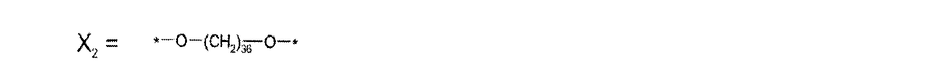 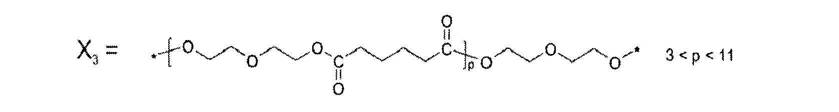 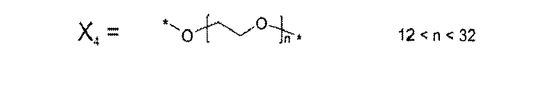 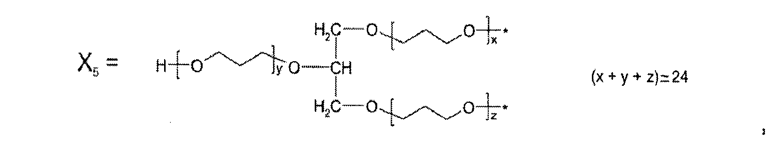 and
